# EUROPÄISCHE PATENTANMELDUNG

(11) **EP 2 465 917 A1**
(43) Veröffentlichungstag der Anmeldung: **20.06.2012**
(21) Anmeldenummer: 10195396.6
(22) Anmeldetag: 16.12.2010
(51) Int. Cl.: C11D 3/382, A61K 8/55, C11D 3/00

(54) **Weichmacher für Textilien**

(71) Anmelder: Cognis IP Management GmbH, 40589 Düsseldorf (DE)
(72) Erfinder: Falkowski, Jürgen, 40789 Monheim (DE); Behler, Ansgar, Dr., 46240 Bottrop (DE); Brabenheim, Monika, 46240 Bottrop (DE)

(57) **Zusammenfassung**

Beschrieben werden Textilbehandlungsmittel, enthaltend hydroxyliertes Lecithin, mindestens einen Dispergator, vorzugsweise eine Alkyl(oligo)glycosid, mindestens ein Wachs und Wasser sowie optional weitere Hilfs- und Zusatzstoffe.

## Beschreibung

Die vorliegende Anmeldung betrifft Textilbehandlungsmittel, die hydroxyliertes Lecithin enthalten und deren Verwendung zur Vermittlung von Weichgriff bei Textilien oder keratinischen Fasern, insbesondere Haaren.

Wasch- und Reinigungsmittel werden in großen Mengen hergestellt. Hauptanwendung ist die Reinigung von textilen Flächengebilden und harten Oberflächen im Haushalt und gewerblichen Bereich.

Sowohl bei der Herstellung und Verarbeitung von textilen Flächengebilden, als auch beim Waschen in häuslichen Bereich verlieren die Textilien hierbei ihren ursprünglichen, meist weichen, angenehmen Griff. Dies liegt beispielsweise daran, dass die ursprünglichen Fett- oder Wachsschichten, die sich beispielsweise auf Naturfasern wie Baumwolle oder Schafwolle befinden, vor der industriellen Weiterverarbeitung in mehreren Waschstufen abgelöst werden müssen. Daher werden auf industriell hergestellten Textilien in der letzten Verarbeitungsstufe nochmals so genannte Weichmacher appliziert. Dies kann - wie dem Fachmann bekannt ist - durch Zwangsapplikation (Foulardverfahren) oder auch im Ausziehverfahren erfolgen. Im häuslichen Bereich müssen diese Textilien zur Entfernung von Anschmutzungen häufig gewaschenen werden. Hierbei werden immer tensidhaltige Wasch- und Reinigungsmittel, die sowohl in fester, meist als Pulver oder Granulat, oder in flüssiger Form eingesetzt.

Dies hat zur Folge, dass der ursprünglich auf dem Textil applizierte Weichmacher entfernt wird und sich der Griff verschlechtert. Weiterhin kommt es während des Waschprozesses zur Ablagerung von alkali- oder erdalkalihaltigen Rückständen, beispielsweise aus dem Buildersystem, auf den Fasern, die das Gewebe hart und steif machen. Daher wird im häuslichen Bereich häufig noch im letzten Spülgang der Waschmaschinen eine flüssige Weichmacherdispersion zugegeben.

Diese Weichmacherdispersion lagert sich dann fein verteilt auf den feuchten Geweben in der Maschine an und bleibt nach dem Trocknen und Bügeln auf den Textilien erhalten. Dies führt dann zu einem weichen, angenehmen Griff auf den trockenen Textilien, der den Tragekomfort der Textilien oder auch die Anwendung, beispielsweise von Hand- oder Badetüchern, verbessert. Häufig werden dem Weichmacher auch verschiedene Parfümöle beigemischt, die dann beispielsweise für einen frischen Geruch der gewaschenen Textilien sorgen. Weiterhin ist es möglich diese Weichmacher auf Tücher zu applizieren, die dann im Trockner bei erhöhter Temperatur den Weichmacher auf die trocknenden Textilien transferieren. Bei den im Haushaltsbereich eingesetzten Weichmachern, handelt es sich meist um Produkte, die auf Basis von Amin- und Fettsäureverbindungen hergestellt werden.

Zur Verbesserung der Substantivität, insbesondere auf Baumwolle, enthalten diese Produkte meist eine kationische Ladung, wie beispielsweise eine quaternäre Ammoniumverbindung. Diese Ladung bewirkt darüber hinaus auch, insbesondere bei Textilien aus synthetischem Material, eine Verbesserung der antistatischen Eigenschaften der trockenen Gewebe. Die bekanntesten Vertreter dieser Produktklasse sind die so genannten Esterquats, die beispielsweise in einem Zweistufenverfahren über die Veresterung von Triethanolamin mit langkettigen Fettsäuren und anschließender Quaternisierung mit Dimethylsulfat hergestellt werden. Weiterhin werden - insbesondere in den Textilfabriken - Aminolyse- oder Amidierungsprodukte aus ein- oder mehrwertigen Aminoalkoholen mit Triglyceriden oder Fettsäuren eingesetzt. Bekannt ist auch das Distearyldimethylammoniumchlorid, welches früher häufig in Haushaltsweichmachern eingesetzt wurde, heute aber in fast allen Ländern aufgrund der besseren biologischen Abbaubarkeit durch Esterquats ersetzt wurde. Eine Zusammenfassung der verschiedenen Textilweichmacher befindet sich beispielsweise in dem Artikel "Softeners in Textile Processing" von Falkowski/Wahle, erschienen 2002 in der Zeitschrift Rev. Prog. Color 32 (2002). Eine gute Zusammenstellung von haushaltsnahen Eigenschaften dieser Inhaltsstoffe befindet sich beispielsweise auch in dem Handbuch Wasch- und Reinigungsmittel von Klaus Henning, erschienen 2006 im Verlag VCI.

Wie oben erwähnt sind zur Herstellung der heute üblichen Weichmacherverbindungen eine oder mehrere Prozessschritte erforderlich. Hierbei werden auch Chemikalien eingesetzt, deren Verwendung erhöhte sicherheitstechnische Anforderungen erfordern und die nicht aus nachwachsenden Rohstoffen herstellbar sind, wie beispielsweise Polyamine oder Quaternisierungschemikalien wie Dimethylsulfat. Weiterhin führen diese Arten von Weichmachern zu einer starken Hydrophobisierung der Oberfläche, das heißt die Saugfähigkeit der Gewebe wird verschlechtert, was beispielsweise bei Hand- oder Badetüchern unerwünscht ist.

Aufgabe der vorliegenden Erfindung war es daher, ein Produkt zu entwickeln, welches verbesserte sensorische Eigenschaften auf textilen Flächengebilden erzeugen und das gleichzeitig auf Basis von nachwachsenden Rohstoffen hergestellt werden kann.

Lecithin ist ein bekanntes Naturprodukt, welches aus tierischen oder pflanzlichen Quellen gewonnen werden kann. Im Allgemeinen enthält Lecithin eine unpolare und eine polare Einheit im Molekül. Weiterhin enthält das Molekül geladene Teile, die im neutralen pH-Bereich eine nach außen neutrale Gesamtladung bewirken, im sauren pH-Bereich ist das Molekül schwach kationisch. Man kann daher Lecithine auch als Zwitterionen oder innere Salze bezeichnen. Aufgrund der der zwei unterschiedlich polaren Gruppen, sind die Moleküle auch oberflächenaktiv. Die unpolare Einheit bewirkt hier wahrscheinlich einen weichmachenden Effekt auf weichen Oberflächen, wie beispielsweise textilen Flächengebilden. Die kationische Ladung im sauren Bereich kann die Substantivität auf beim Waschen in der Regel negativ geladenen Baumwolloberflächen verbessern. Daher war es auch nicht überraschend, dass Lecithine in der Literatur als Weichmacher für Fasern oder auch Haaren beschrieben werden.

Beispielsweise beschreibt US 4808320 (Fabric softening Compositions based on lecithin and methods for making and using same; Jacques et al.; 1989, Colgate-Palmolive Company) die direkte Anwendung von verschieden konzentrierten Lecithindispersionen als Weichmacher für Haushaltswäschen. US 816170 (Stable aqueous fabric softening compositions based on lecithin, saponin and sorbic acid and methods for making and using same; Jacques et al., 1989 Colgate-Palmolive Company) beschreibt Kombinationen von Lecithinen mit Saponinen als Weichmacher für Haushaltswäschen. US 4642919 (Textile treating compositions and methods, Yi-Chang Fu, 1987 The Procter&Gamble Company) beschreibt Kombinationen von Lecithinen mit kationischen Weichmachern.

Die beschriebenen Verfahren weisen einige Nachteile auf, die auch der Grund sind, warum diese Produkte bislang kommerziell nicht erhältlich sind. Reines Lecithin ist als Naturprodukt eine hochviskose, bräunliche Flüssigkeit, die sich nur schwer verarbeiten lässt. Die Herstellung von reinen Lecithindispersionen bzw. Abmischungen dieser Dispersionen führt daher ebenfalls zu instabilen, insbesondere bei höheren Konzentrationen schwer zu dosierenden Produkten. Darüber hinaus sind Produkte auch für Wasch- und Reinigungsmittelmarkt zu teuer. Die Anwendungen sind daher bislang auf den Ernährungs- Nahrungsmittelergänzungsbereich beschränkt geblieben.

Es wurde nun gefunden, dass die Verwendung von hydroxyliertem Lecithin die oben geschilderten Probleme lösen kann.

Ein erster Gegenstand der vorliegenden Anmeldung betrifft daher Textilbehandlungsmittel, enthaltend hydroxyliertes Lecithin, mindestens einen Dispergator, mindestens ein Wachs und Wasser.

Die Herstellung der hydroxylierten Lecithine kann beispielsweise durch die Behandlung des Lecithins mit Wasserstoffperoxid erfolgen. Hierbei wird die Doppelbindung der ungesättigten Fettsäure im unpolaren Molekül oxidiert und anschließend mit Wasser ringgeöffnet. Ein Herstellprozess für solche Lecithinderivate und hydroxylierte Lecithine im Sinne der vorliegenden Lehre werden beispielsweise in der US 2,621,133 oder der US 6,638,544 beschrieben. Die US 2,621,133 offenbart die Verwendung von hydroxyliertem Lecithin mit Stärke oder Stärkederivaten als Schlichtemittel, erwähnt aber nicht die Verwendung als Textilweichmacher.

Diese hydroxylierten Lecithine sind wesentlich heller und oxidationsstabiler als die nicht-hydroxylierten Lecithine. Hierbei können grundsätzlich alle Arten von pflanzlichen oder tierischen Lecithinen verwendet werden, vorzugsweise aber solche auf Basis von Hühnereiweis, Raps- oder Sojaöl gewonnenen Qualitäten. Als Beispiel für hydroxyliertes Lecithin wäre nur beispielsweise das kommerziell erhältliche Produkt der Fa. Solae Company, Solec^{®} A, zu nennen.

Es wurde gefunden, dass diese hydroxylierten Lecithine a) in Kombination mit Dispergatoren b) bei Raumtemperatur (= 21 °C) feste Wachse c) in Wasser dispergieren können. Diese erfindungsgemäßen Dispersionen sind hellfarbig, stabil und dünnflüssig, was insbesondere für die Dosierung und Verteilung in einer haushaltsüblichen Waschmaschine wichtig ist. Die sensorischen Eigenschaften der mit diesen Produkten behandelten Textilien sind besser als die mit üblichen Textilweichmachern, z.B. von quaternierten Ammoniumverbindungen behandelten Gewebe. Die Mittel sind Flüssigkeiten, die Feststoffe dispergiert enthalten. Eine Dispersion ist bekanntlich ein heterogenes Gemisch aus mindestens zwei Stoffen, die sich nicht oder kaum ineinander lösen oder chemisch miteinander verbinden. In der Regel handelt es sich dabei um Kolloide. Dabei wird ein Stoff (dispergierte Phase, disperse Phase, innere Phase oder Nebenphase) möglichst fein in einem anderen Stoff (Dispersionsmittel, Dispersionsmedium, Dispergens, kontinuierliche Phase, äußere Phase oder Hauptphase) verteilt. Die einzelnen Phasen können dabei deutlich von einander abgegrenzt werden.

Erfindungsgemäß weisen die Textilbehandlungsmittel eine kinematische Viskosität (gemessen bei 20 °C mit einem Brookfield-Viskosimeter, Spindel 3, 20 rpm) von vorzugsweise kleiner/gleich 1000 mPa s auf. Insbesondere bevorzugt sind Mittel deren kinematische Viskosität (gemessen bei 20 °C mit einem Brookfield-Viskosimeter, Spindel 3, 20 rpm) zwischen 15 und 250 mPa s, vorzugsweise zwischen 25 und 150 mPa s, und insbesondere zwischen 45 und 110 mPa s liegt.

Als Dispergatoren b) können nichtionische, anionische oder kationische Produkte eingesetzt werden.

Nichtionische Dispergatoren können sein: Fettalkoholpolyglycolethern, Alkylphenolpolyglycolethern, Fettsäure-polyglycolestern, Fettsäureamidpolyglycolethem, Fettaminpolyglycolethern, alkoxylierten Triglyceriden, (Hydroxy)Mischethem bzw. Mischformalen, Alk(en)yloligoglykosiden, Fettsäure-N-alkylglucamiden, Proteinhydrolysaten, Polyolfettsäureestern, Zuckerestern, Sorbitanestern, Polysorbaten und Aminoxiden.

Besonders bevorzugte nichtionische Dispergatoren sind die Alkyl- und/oder Alkenyloligoglykoside zu nennen, die der Formel (I) folgen, R¹O-[G]ₚ in der R¹ für einen linearen oder verzeigten, gesättigten oder ungesättigten Alkyl- und/oder Alkenylrest mit 4 bis 22 Kohlenstoffatomen, G für einen Zuckerrest mit 5 oder 6 Kohlenstoffatomen, vorzugsweise eine Glycosidrest und p für Zahlen von 1 bis 10 und vorzugsweise von 1 bis 6 steht.

Diese Alkyl(ologo)glycoside können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C8-C10 (DP = 1 bis 3), die als Vorlauf bei der destillativen Auftrennung von technischem C8-C18-Kokosfettalkohol anfallen und mit einem Anteil von weniger als 6 Gew.-% C12-Alkohol verunreinigt sein können sowie Alkyloligoglucoside auf Basis technischer C9/11-Oxoalkohole (DP = 1 bis 3). Der Alkyl- bzw. Alkenylrest R kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C12/14-Kokosalkohol mit einem DP von 1 bis 3.

Weitere typische Beispiele für geeignete Stoffe, die alternativ die Komponente (b) bilden, sind anionische Tenside, die ausgewählt sind aus der Gruppe, die gebildet wird Seifen, Alkylbenzolsulfonaten, Alkansulfonaten, Olefinsulfonaten, Alkylethersulfonaten, Glycerinethersulfonaten, [alpha]-Methylestersulfonaten, Sulfofettsäuren, Alkylsulfaten, Alkylethersulfaten, Glycerinethersulfaten, Hydroxymischethersulfaten, Monoglycerid(ether)sulfaten, Fettsäureamid-(ether)sulfaten, Mono- und Dialkylsulfosuccinaten, Mono- und Dialkylsulfosuccinamaten, Sulfotriglyceriden, Amidseifen, Ethercarbonsäuren und deren Salzen, Fettsäureisethionaten, Fettsäuresarcosinaten, Fettsäuretauriden, N-Acylaminosäuren, Alkyloligoglucosidsulfaten, Proteinfettsäurekondensaten und Alkyl(ether)phosphaten.

Hier haben sich insbesondere Alkylethersulfate als besonders vorteilhaft erwiesen, die vorzugsweise der Formel (II) folgen: RO(CH₂CH₂O)ₙ-SO₃X (II), in der R für einen linearen oder verzweigten, aliphatischen Alkyl- und/oder Alkenylrest mit 6 bis 22, vorzugsweise 12 bis 18 Kohlenstoffatomen, n für Zahlen von 1 bis 10, vorzugsweise 2 bis 5 und X für ein Alkali- und/oder Erdalkalimetall, Ammonium, Alkylammonium, Alkanolammonium oder Glucammonium steht. Typische Beispiele für Alkylethersulfate, die im Sinne der Erfindung Anwendung finden können, sind die Sulfatierungsprodukte von Anlagerungsprodukten von durchschnittlich 1 bis 10 und insbesonder 2 bis 5 Mol Ethylenoxid an Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen. Die Sulfatierungsprodukte können vorzugsweise in Form ihrer Alkalisalze und insbesondere ihrer Natriumsalze eingesetzt werden.

Bei den nichtionischen Dispergatoren können vorzugsweise Zuckertenside und besonders bevorzugt Tenside aus der Klasse der Alkylpolyglucoside verwendet werden. Der Zusatz dieser Tenside führt zu einer erwünschten Absenkung der Viskosität der Dispersionen und verbessert gleichzeitig die Stabilität der Dispersionen. Bei den anionischen Tensiden werden vorzugsweise Tenside aus der Klasse der Fettalkoholsulfate und besonders hier besonders bevorzugt langkettige Fettalkoholsulfate, wie beispielsweise Natriumpalmitylsulfat, Natriumstearylsulfat oder Mischungen aus langkettigen Fettalkoholsulfaten, eingesetzt. Weiterhin können zur Einstellung der Viskosität auch kleine Mengen an organischen oder anorganischen Salzen, wie beispielsweise Natriumsulfat, Natriumsulfit, Natriumcumolsulfonat oder Harnstoff, eingesetzt werden.

Als Wachse c) können grundsätzlich alle Arten von bei Raumtemperatur (21 °C) festen Produkten eingesetzt werden, die auf der Basis von petrochemischen oder natürlichen Rohstoffen hergestellt wurden, verwendet werden.

Zu den Wachsen, die auf Basis von petrochemischen Grundstoffen hergestellt werden, zählen beispielsweise einfache Kohlenwasserstoffverbindungen, wie Paraffinwachse, oder auch durch Polymerisation hergestellte Homopolymere, wie Polyethylene, Polyvinylacetate, Polyacrylate, oxidierte Homopolymere, wie beispielsweise oxidierte Polyethylene, Copolymere auf Basis von Ethylen-Acrylsäure, Ethylen-Propylen-Maleinsäureanhydrid, Ethylen-Vinylacetat oder mikronisierte Polyethylenwachse. Der Schmelzpunkt dieser Verbindungen liegt vorzugsweise zwischen 40 °C und 160 °C, besonders bevorzugt zwischen 60 °C und 140 °C. Als Beispiele wären hier einige Handelsprodukte - wieder ohne Anspruch auf Vollständigkeit zu nennen: AC330, AC 175, AC 5120, ACumist A12 von Honeywell, Licowax PE 130, Licowax PED 521, Licowax PED 192 von der Fa. Clariant, Luwax OA3-Wachs von der Fa. BASF. Weiterhin können auch Handelsprodukte bei denen das Polyethylenwachs bereits mit anionischen, nichtionischen oder kationischen Emulgatoren bzw. Dispergatoren in wässrigen Lösungen dispergiert ist, wie beispielsweise das Polyquart^{®} CCE der Fa. Cognis, verwendet werden.

Bevorzugt werden aber, wie in der Aufgabenstellung auch beschrieben, Wachse auf Basis von nachwachsenden Rohstoffen, also insbesondere Wachse, die beispielsweise durch Veresterung, Umesterung, Veretherung oder Amidierung herstellbar sind, verwendet. Bei diesen Verfahren können beispielsweise längerkettige Fettsäuren, Fettalkohole und Fettamine, vorzugsweise mit C-Ketten von C6 - C22 eingesetzt werden. Weiterhin ist es aber auch möglich langkettige Fettsäuren, Fettsäurechloride, Fettalkohole oder Fettamine, mit kurzkettigen, ein- oder mehrwertigen Carbonsäuren, Alkoholen oder Aminverbindungen, Aminocarbonsäuren, Hydroxylaminverbindungen zu kombinieren, also beispielsweise Ethanol, n-Butanol, Ethylenglykol, Sorbitan, Diethylenglykol, Glycerin, Triethanolamin, Aminoethylethanolamin. Der Schmelzpunkt dieser Wachse liegt vorzugsweise zwischen 20 °C und 120 °C, besonders bevorzugt zwischen 30 °C und 80 °C. Als Beispiele - ohne Anspruch auf Vollständigkeit - wären zu nennen: Butylstearat, Cetylpalmitat, Ethylenglykoldistearat, Glycerinmonostearat, Stearylcitrat, Triethanolamindistearat, Stearylglutamat, Di-n-Cetylether und Di-n-Stearylether. Darüber hinaus können auch direkt Fettsäuren, Fettalkohole oder Triglyceride, wie beispielsweise Stearinsäure, Stearylalkohol, gehärtetes Palmfett oder natürlich vorkommende Kohlenwasserstoffverbindungen, wie beispielsweise Squalane eingesetzt werden.

Typische Beispiele für kationische Dispergatoren sind insbesondere Tetraalkylammoniumverbindungen, wie beispielsweise Dimethyldistearylammoniumchloridldistearylammoniumchlorid oder Hydroxyethyl / Hydroxycetyl Dimmonium Chloride oder sog. Esterquats.

Hierbei handelt es sich beispielsweise um quaternierte Fettsäuretriethanolaminestersalze bzw. deren alkoxylierten Derivaten. Neben den quaternierten Fettsäuretriethanolaminestersalzen kommen als Esterquats ferner auch quaternierte Estersalze von Fettsäuren mit Diethanolalkylaminen) in Betracht. Als weitere Gruppe geeigneter Esterquats sind quaternierten Estersalze von Fettsäuren mit 1,2-Dihydroxypropyldialkylaminen zu nennen. Schliesslich kommen als Esterquats noch Stoffe in Frage, bei denen die Ester- durch eine Amidbindung ersetzt ist. Derartige Amidesterquats sind beispielsweise unter der Marke Incroquat (Croda) im Markt erhältlich.

Beispiele für geeignete amphotere bzw. zwitterionische Tenside sind Alkylbetaine, Alkylamidossine, Aminopropionate, Aminoglycinate, Imidazoliniumbetaine und Sulfossine. Beispiele für geeignete Alkylbetaine stellen die Carboxyalkylierungsprodukte von sekundären und insbesondere tertiären Aminen dar, wie etwa die Carboxymethylierungsprodukte von Hexylmethylamin, Hexyldimethylamin, Octyldimethylamin, De-cyldimethylamin, Dodecylmethylamin, Dodecyldimethylamin, Dodecylethylmethylamin, C12/14-Kokosalkyldimethylamin, Myristyldimethylamin, Cetyldimethylamin, Stearyldimethylamin, Stearylethylmethylamin, Oleyldimethylamin, C16/18-Talgalkyldimethylamin sowie deren technische Gemische. Weiterhin kommen auch Carboxyalkylierungsprodukte von Amidoaminen in Betracht, so zum Beispiel Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Eläostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C8/18-Kokosfettsäure-N,N-dimethylaminopropylamid mit Natriumchloracetat. Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (ÄEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C12/14-Kokosfettsäure, die anschließend mit Natriumchloracetat betainisiert werden.

Besonders bevorzugt als Dispergatoren sind aber Fettalkohole mit mindestens 16 Kohlenstoffatomen oder Polyolester. Bevorzugte Polyolester enthalten als PolyolKomponente Sorbitane, oder vorzugsweise Glycerin, und als Säure Fettsäuren mit 6 bis 22 C-Atomen, wobei insbesondere ungesättigte Fettsäuren bevorzugt sein können. Weiterhin können natürliche oder synthetische Polymerdispergatoren verwendet werden, z.B. Natriumpolyacrylate (Cosmedia^{®} SP der Fa. Cognis) oder Produkte auf Basis von Guar und Xanthan Gum. Weiterhin sind kationisierte Stärke oder Cellulose (z.B. Polyquaternium-10) geeignete Dispergatoren (weitere Handelsprodukte sind z.B. Polymer JR oder das Dehyquart^{®} Guar der Fa. Cognis).

Die Textilbehandlungsmittel enthalten das hydroxylierte Lecithin vorzugsweise in Mengen von 0,1 bis 10 Gew.-%, vorzugsweise von 1 bis 8 und insbesondere von 1,5 bis 5 Gew.-%, bezogen auf das Gesamtgewicht des Mittels. Die Dispergatoren sind in Mengen von 0,1 bis 5 Gew.-%, vorzugsweise von 0,2 bis 2,5 Gew.-% und insbesondere von 0,5 bis 1 Gew.-%, bezogen auf das Gesamtgewicht des Mittels, enthalten und Wachse in Mengen von 1 bis 15 Gew.-%, vorzugsweise von 2 bis 10 Gew.-% und insbesondere von 2,5 bis 5 Gew.-%, bezogen auf das Gesamtgewicht des Mittels.

Die Mittel der vorliegenden Erfindung sind immer wässerig (vorzugsweise wird vollentsalztes Wasser ausgewählt) und enthalten vorzugsweise das Wasser in Mengen von 65 bis 98 Gew.-%, vorzugsweise von 80 bis 95 Gew.-% und insbesondere von 85 bis 95 Gew.-%, bezogen auf das Gesamtgewicht des Mittels. Die Textilbehandlungsmittel können weiterhin noch Zusatzstoffe e), ausgewählt aus der Gruppe organische und/oder anorganische Salze (beispielsweise Natriumchlorid, Natriumsulfat, Natriumcumolsulfonat oder Harnstoff), Emulgatoren, Konservierungsmittel (z.B. Benzoesäure), Farbstoffe, Duftstoffe, Polymere, nicht-wässerige Lösungsmittel und Säuren bzw. Basen enthalten. Letztere werden verwendet, um die wässrigen Mittel auf einen gewünschten pH-Wert einzustellen, der vorzugsweise zwischen 2,0 und 8,0 und insbesondere von 3,0 bis 7,0, wobei ein Wert zwischen 3,0 und 4,0 bevorzugt ausgewählt sein kann.

Die Zusatzstoffe e) sind dann vorzugsweise in Mengen von 0,01 bis 10,0 Gew.-%, vorzugsweise von 0,1 bis 5,0 Gew.-% und insbesondere von 1,0 bis 5,0 Gew.-%, bezogen auf das Gesamtgewicht des Mittels, enthalten.

Die Textilbehandlungsmittel sind vorzugsweise frei von kationischen Textilweichmachern, Stärke und Stärkederivaten, Liposomen und/oder nicht-hydroxyliertem Lecithin. Vorzugsweise können die Mittel frei sein von kationischen Textilweichmachern, die insbesondere ausgewählt sind aus der Gruppe quaternierten Fettsäuretriethanolaminestersalzen, wie sie z.B. konkret in der EP 848 103 A2 beschrieben werden, siehe hier insbesondere die Strukturen gemäß der allgemeinen Formel (I), (II) und (III) in den Ansprüchen der Schrift und gemäß der Beschreibung in auf den Seite 4, Zeile 11 bis Seite 5, Zeile 32.

In einer weiteren Ausführungsform bestehen die Mittel nur aus den Inhaltstoffen a) - d) gemäß der obigen Beschreibung.

Die Herstellung der Mittel erfolgt in an sich bekannte Art und Weise indem die Komponenten vermischt und anschließend unter Rühren erwärmt werden. Die Komponenten werden vorzugsweise auf eine Temperatur oberhalb des Schmelzpunktes der Wachskomponente erwärmt, insbesondere ist damit der Temperaturbereiche von 60 bis 80 °C auszuwählen. Nach ca. 20 bis 40 Minuten entsteht dann eine stabile Dispersion, die abgekühlt und ggf. noch mit anorganischen oder organischen Säuren oder Basen auf einen gewünschten pH-Wert eingesellt wird. Es ist aber auch möglich, in Wasser vordispergierte Wachse, z.B. eine wässerige Polyethylendispersion oder Wachse auf Basis von Carbonsäureestern (z.B. Polyquart^{®} CCE bzw. Plantatex^{®} HCC, beide von Cognis) einzusetzen, die dann direkt mit den anderen Komponenten zusammengerührt werden können.

Die Mittel bzw. die hydroxylierten Lecithine werden vorzugsweise zur Vermittlung von Weichgriff bei textilen Fasern und daraus hergestellten Flächengebilden, insbesondere solchen aus Wolle oder Baumwolle, aber auch für keratinische Fasern (z.B. Haaren) verwendet. Die Verwendung schließt aber auch Textilien oder Fasern ein, die synthetische Fasern enthalten ("Mischgewebe") oder aus synthetischen Fasern bestehen. Somit eignet sich hydroxyliertes Lecithin vorzugsweise zur Herstellung von Waschmitteln, Wäschenachbehandlungsmitteln, aber auch zur Herstellung von kosmetischen Mitteln, insbesondere für Shampoos.

Die Mittel gemäß der vorliegenden Beschreibung können z.B. als Textilbehandlungsmittel in einem Waschprozess für Textilien verwendet werden, wobei die Mittel, vorzugsweise in einer Haushaltswaschmaschine mit den Textilien in Kontakt gebracht werden. Dabei kann das hydroxylierte Lecithin, vorzugsweise in Form eines wachshaltigen Mittels wie oben beschrieben, vor, während, oder nach dem eigentlichen Waschgang zugegeben werden. Dabei kann es von Vorteil sei, während des Inkontaktbringens die Temperatur des Wassers zu erhöhen, z.B. auf Werte zwischen 30 und 60 °C.

### Beispiele

Als Beispiel wurden 4 verschiedene Formulierungen hergestellt. Zur Herstellung der lecithinhaltigen Dispersionen wurden alle Komponenten in einem Becherglas vorgelegt und unter Rühren auf 75 °C aufgeheizt. Bei dieser Temperatur wurden 30 min nachgerührt und anschließend wieder unter Rühren auf Raumtemperatur gekühlt. Die Viskosität wurde danach mit einem Brookfield-Viskosimeter (20 °C, Spindel 3, 20 rpm) bestimmt.

Die so hergestellten Dispersionen hatten folgende Zusammensetzung (Angaben immer in Gew.-%, Rest auf 100 Gew.-%: Wasser):

| | **Formulierung** | **Formulierung** | **Formulierung** | **Formulierung** |
|---|---|---|---|---|
| | **1** | **2** | **3** | **4** |
| Lanette^{®} O | 4 | | | |
| Dehymuls^{®} SMS | | 4 | | |
| Cutina^{®} GMS-V | | | 4 | 4 |
| Lanette^{®} E | 1 | 1 | 1 | 1 |
| Hydroxyliertes | 2 | 2 | 2 | 2 |
| Sojalecithin | | | | |
| Glucopon^{®} 425 | | | 0,5 | |
| N/NH | | | | |
| Glucopon^{®} 600 | 0,5 | 0,5 | | 0,5 |
| CS UP | | | | |
| Harnstoff | | | 0,1 | 0,1 |
| Benzoesäure | 0,5 | 0,5 | 0,5 | 0,5 |
| pH | 3-4 | 3-4 | 3-4 | 3-4 |
| Viskosität [mPas] | 50 | 25 | 100 | 50 |

Bei Lanette^{®} O handelt es sich um ein Gemisch aus Palmityl- und Stearylalkohol der Fa. Cognis, Dehymuls^{®} SMS ist der Sorbitanmonostearat der Fa. Cognis, Cutina^{®} GMS-V ist ein Glycerinmonostearat der Fa. Cognis, Lanette E ist ein Natriumpalmitylsulfat der Fa. Cognis, das hydroxilierte Sojalecithin war ein Produkt der Fa. Solae Company mit der Bezeichnung Solec^{®} A, Glucopon^{®} 425 N/NH bzw. Glucopon^{®} 600 CS UP sind Alkylpolyglucoside der Fa. Cognis. Handelsübliche Benzoesäure (Fa. Sigma/Aldrich) wurde beispielhaft als Konservierungsmittel verwendet, sowie Harnstoff (Fa. Sigma /Aldrich) zur Einstellung der Viskosität.

Zum Vergleich der damit zu erzielenden Weichheitsleistung wurde eine Dispersion mit 15 Gew.% eines handelsüblichen kationischen Weichmachers (Dehyquart^{®} AU 46 der Fa. Cognis) hergestellt. Mit diesen Produkten wurden Waschversuche durchgeführt und die gewaschenen Textilien wurden mit einer objektiven Methode zur Bewertung der Weichheit/Glätte gegeneinander verglichen.

### 1) Waschversuche mit verschiedenen Konzentrationen an Esterquat.

Je 2000 g Ballastwäsche aus Baumwolle pro Maschine wurden zur Entfernung der Appretur dreimal bei 95 °C mit je 75 g ECE-2 (Lieferant: Fa. wfk / Krefeld) vorgewaschen und noch dreimal bei 95 °C ohne Waschmittel gespült. Danach wurden diese Wäscheposten bei 40 °C mit ECE-2 gewaschen und in den Spülgang jeweils unterschiedliche Mengen an Dehyquart^{®} AU 46 zugegeben. Danach wurde die Wäsche getrocknet und aus einem kleinen Teil wurde kleine rechteckige Streifen ausgestanzt. Diese Streifen wurden klimatisiert und anschließend mittels mit einer Zwickmaschine zur Aufnahme von Kraft-Weg Diagrammen durch ein spezielle Anordnung von metallischen Stangen gezogen.

Je geringer die Kraft zum Durchziehen der Streifen ist, desto glatter/weicher und biegsamer ist das textile Flächengebilde. Diese Methode ist vergleichbar mit bekannten Methoden zur Messung der Nass- und Trockenkämmbarkeit von Haaren. Vorher durchgeführte Versuche zur Methodenvalidierung zeigten eine gute Übereinstimmung mit sensorischen Daten, die von menschlichen Probanden auf einer monadischen Skala zur Bewertung von Glätte, Weichheit und Biegsamkeit ermittelt wurden. Folgende Werte wurden hierbei ermittelt:

| | **Maschine 1** | **Maschine 2** | **Maschine 3** | **Maschine 4** | **Maschine 5** |
|---|---|---|---|---|---|
| Menge der 15 Gew% ige Dispersion Dehyquart^{®} AU 46 | -(nur Spülung mit Wasser) | 2,4 g | 12 g | 24 g (Standard-menge) | 36 g |
| Kraft [N] | 1,66 +/-0,13 | 1,48 +/- 0,14 | 1,22 +/- 0,06 | 1,00 +/- 0,08 | 0,83 +/- 0,16 |

Man sieht, dass mit steigender Menge an kationischem Weichmacher die Kraft zum Durchziehen der Streifen erniedrigt wird, das heißt die Textilien werden - wie zu erwarten war - glatter/weicher und biegsamer.

### 2) Waschversuche mit den lecithinhaltigen Wachsdispersionen

Analog zu denn unter 1) beschriebenen Waschversuchen wurden die oben beschriebenen lecithinhaltigen Wachsdispersionen in den letzten Spülgang der Maschine gegeben. Zum Vergleich wurde nur mit Wasser und der Standardmenge an kationischem Weichmacher gespült. Nach analoger Behandlung wurden die folgenden Daten ermittelt:

| | **Maschine** | **Maschine** | **Maschine** | **Maschine** | **Maschine** | **Maschine** |
|---|---|---|---|---|---|---|
| | **1** | **2** | **3** | **4** | **5** | **6** |
| Menge Weichmac her | -(nur Spülung mit Wasser) | 24 g Esterquat, 15%ig (Standard-menge) | 48 g Formulierung 1 7%ig | 48 g Formulierung 2 7 %ig | 48 g Formulierung 3 7 %ig | 48 g Formulierung 4 7%ig |
| Kraft [N] | 1,94 +/- 0,29 | 1,08 +/- 0,06 | 1,09 +/- 0,06 | 1,13 +/- 0,04 | 0,96 +/- 0,05 | 0,96 +/- 0,01 |

Man sieht, dass alle lecithinhaltigen Wachsdispersionen bei gleicher Konzentration ähnliche niedrige Werte für die Kraft erreichen wie die esterquathaltigen Dispersion. Damit erreichen die erfindungsgemäßen lecithinhaltigen Wachsdispersionen ähnliche, teilweise sogar bessere Leistungen in Bezug auf Glätte, Weichheit und Biegsamkeit von textilen Flächengebilden.

## Patentansprüche

1. Textilbehandlungsmittel, enthaltend
a) hydroxyliertes Lecithin
b) mindestens einen Dispergator
c) mindestens ein Wachs und
d) Wasser

2. Textilbehandlungsmittel nach Anspruch 1, **dadurch gekennzeichnet, dass** als Dispergatoren Verbindungen der Formel (I) R¹-O-[G]ₚ ausgewählt sind, wobei R¹ für einen lineare oder verzweigten, gesättigten oder ungesättigten Alkenyl- oder Alkylrest mit 4 bis 22 C-Atomen steht, G einen Glycosidrest bedeutet und p Werte von 1 bis 6 annehmen kann.

3. Textilbehandlungsmittel nach mindestens einem der Ansprüche 1 bis 2, **dadurch gekennzeichnet, dass** als Wachse solche auf Basis nachwachsender Rohstoffe ausgewählt sind.

4. Textilbehandlungsmittel nach mindestens einem der Ansprüche 1 bis 3, **dadurch gekennzeichnet, dass** als Wachse Fettalkohole mit mindestens 16 Kohlenstoffatomen oder Polyolester ausgewählt sind.

5. Textilbehandlungsmittel nach mindestens einem der Ansprüche 1 bis 4, **dadurch gekennzeichnet, dass** das Lecithin für die Hydroxylierung ausgewählt ist aus Soja-, Rapsöl- oder Hühnereilecithin.

6. Textilbehandlungsmittel nach mindestens einem der Ansprüche 1 bis 5, **dadurch gekennzeichnet, dass** die Mittel das hydroxylierte Lecithin in Mengen von 0,1 bis 10 Gew.-%, vorzugsweise von 1 bis 8 und insbesondere von 1,5 bis 5 Gew.-%, bezogen auf das Gesamtgewicht des Mittels, enthalten.

7. Textilbehandlungsmittel nach mindestens einem der Ansprüche 1 bis 6, **dadurch gekennzeichnet, dass** die Mittel Dispergatoren in Mengen von 0,1 bis 5 Gew.-%, vorzugsweise von 0,2 bis 2,5 Gew.-% und insbesondere von 0,5 bis 1 Gew.-%, bezogen auf das Gesamtgewicht des Mittels, enthalten.

8. Textilbehandlungsmittel nach mindestens einem der Ansprüche 1 bis 7, **dadurch gekennzeichnet, dass** die Mittel Wachse in Mengen von 1 bis 15 Gew.-%, vorzugsweise von 2 bis 10 Gew.-% und insbesondere von 2,5 bis 5 Gew.-%, bezogen auf das Gesamtgewicht des Mittels, enthalten.

9. Textilbehandlungsmittel nach mindestens einem der Ansprüche 1 bis 8, **dadurch gekennzeichnet, dass** die Mittel Wasser in Mengen von 65 bis 98 Gew.-%, vorzugsweise von 80 bis 95 Gew.-% und insbesondere von 85 bis 95 Gew.-%, bezogen auf das Gesamtgewicht des Mittels, enthalten.

10. Textilbehandlungsmittel nach mindestens einem der Ansprüche 1 bis 9, **dadurch gekennzeichnet, dass** die kinematische Viskosität (gemessen bei 20 °C mit einem Brookfield-Viskosimeter, Spindel 3, 20 rpm) zwischen 15 und 250 mPa s, vorzugsweise zwischen 25 und 150 mPa s, und insbesondere zwischen 45 und 110 mPa s liegt.

11. Textilbehandlungsmittel nach mindestens einem der Ansprüche 1 bis 10, **dadurch gekennzeichnet, dass** es weiterhin noch Zusatzstoffe e), ausgewählt aus der Gruppe organische und/oder anorganische Salze, Emulgatoren, Konservierungsmittel, Farbstoffe, Duftstoffe, Polymere, nicht-wässerige lösungsmittel und Säuren bzw. Basen enthält.

12. Textilbehandlungsmittel nach mindestens einem der Ansprüche 1 bis 11, **dadurch gekennzeichnet, dass** die Mittel die Zusatzstoffe e) in Mengen von 0,01 bis 10,0 Gew.-%, vorzugsweise von 0,1 bis 5,0 Gew.-% bezogen auf das Gesamtgewicht des Mittels, enthalten.

13. Textilbehandlungsmittel nach mindestens einem der Ansprüche 1 bis 12, **dadurch gekennzeichnet, dass** die Mittel frei sind von kationischen Textilweichmachern, Liposomen und/oder nicht-hydroxyliertem Lecithin.

14. Textilbehandlungsmittel, bestehend aus den Inhaltstoffen a) - d) gemäß der Beschreibung in Anspruch 1.

15. Verwendung von Mittel gemäß Anspruch 1, zur Weichmachung von Textilien oder Keratin-haltigen Fasern.

16. Verwendung von hydroxyliertem Lecithin zur Herstellung von Waschmitteln, Wäschenachbehandlungsmitteln, oder kosmetischen Zubereitungen, vorzugsweise von Shampoos.

17. Verfahren zum Weichmachen von Textilien, **dadurch gekennzeichnet, dass** ein Mittel gemäß Anspruch 1 in einem Waschprozess, vorzugsweise mit Hilfe einer Haushaltswaschmaschine, mit den Textilien in Kontakt gebracht wird.

18. Verwendung von hydroxyliertem Lecithin als Weichmachern für Textilfasern oder keratinische Fasern.
